# EUROPEAN PATENT APPLICATION

(11) **EP 4 840 483 A1**
(43) Date of publication of application: **18.11.2026**
(21) Application number: 25738805.8
(22) Date of filing: 09.01.2025
(51) Int. Cl.: A61M 1/00, A61F 2/04, A61F 2/856

(54) **URETERAL STENT**

(30) Priority: 10.01.2024 JP 2024001656
(71) Applicant: Nipro Vascular Corporation, Nagoya-shi, Aichi 460-0008 (JP); PUBLIC UNIVERSITY CORPORATION NAGOYA CITY UNIVERSITY, Nagoya-shi, Aichi 467-8601 (JP)
(72) Inventor: TAMURA, Mizuki, Seto-shi, Aichi 489-0976 (JP); OHASHI, Natsumi, Seto-shi, Aichi 489-0976 (JP); HIRATSUKA, Shinsuke, Seto-shi, Aichi 489-0976 (JP); OKADA, Shinsuke, Ichikawa-shi, Chiba 272-0103 (JP); INOUE, Takaaki, Kobe-shi, Hyogo 650-0012 (JP); HAMAMOTO, Shuzo, Nagoya-shi, Aichi 467-8601 (JP)
(74) Representative: Epping - Hermann - Fischer
(86) International application number: PCT/JP2025/000525
(87) International publication number: WO 2025/150538

(57) **Abstract**

A ureteral stent (1A) includes a tubular body (2). The tubular body (2) includes a first curved part (31) configured to be disposed in a kidney (91), a second curved part (32) configured to be disposed in a bladder (93), and an extension part (30) extending between the first curved part (31) and the second curved part (32). A first material that is an X-ray opaque material is contained, in a first proportion, in at least part of the target curved part. A color of at least part of the target curved part is a first color. A second material that is an X-ray opaque material is contained, in a second proportion, in the extension part. A color of the extension part is a second color. An X-ray transmittance of the target curved part is different from an X-ray transmittance of the extension part.

## Description

### Technical Field

The present invention relates to a ureteral stent.

### Background art

A ureteral stent is known that is used for preventing blockage of the ureter joining the kidney and the bladder. A ureteral stent disclosed in Patent Literature 1 includes an intermediate part and hooks provided at both end parts of the intermediate part. The intermediate part is disposed in the ureter. Further, the hook at one end part of the intermediate part is disposed in the kidney (renal pelvis), and the hook at the other end part of the intermediate part is disposed in the bladder. The placing of the ureteral stent in the body is performed by checking, using a cystoscope, whether the hook at the other end portion of the intermediate part has been disposed at an appropriate position inside the bladder.

### Citation List

### Patent Literature

Patent Literature 1: JP H02-052646 A

### Summary of Invention

There is a demand to place a ureteral stent in the body while checking the position of the ureteral stent under X-ray radioscopy. However, with the ureteral stent of the related art, checking the position thereof under X-ray radioscopy is difficult. Thus, there are cases in which it is not possible to place the ureteral stent at the appropriate position in the body under X-ray radioscopy.

An object of the present invention is to provide a ureteral stent with which it is possible to place the ureteral stent at an appropriate position in the body under X-ray radioscopy.

A ureteral stent according to an aspect of the present invention includes a tubular body extending between one end part and another end part. The tubular body includes a first curved part configured to be disposed in a kidney, a second curved part configured to be disposed in a bladder, and an extension part extending between the first curved part and the second curved part. A target curved part is at least one of the first curved part and the second curved part. A first material that is an X-ray opaque material is contained, in a first proportion, in at least part of the target curved part. A color of at least part of the target curved part is a first color. A second material that is an X-ray opaque material is contained, in a second proportion, in the extension part. A color of the extension part is a second color. An X-ray transmittance of the target curved part is different from an X-ray transmittance of the extension part.

A user performing treatment on a patient using the ureteral stent can check, under X-ray radioscopy, the positions of each of the extension part and the target curved part of the ureteral stent inside the body. Further, since the X-ray transmittance of the target curved part and the X-ray transmittance of the extension part are different from each other, the user can easily check, under the X-ray radioscopy, a boundary position between the target curved part and the extension part. Thus, under the X-ray radioscopy, the user can place the ureteral stent in the body in a state in which the target curved part is appropriately disposed in the kidney or the bladder.

In the ureteral stent according to the aspect, the first material may be different from the second material. In this case, by causing the first material and the second material to be different from each other, the ureteral stent can realize the difference in the X-ray transmittances of each of the target curved part and the extension part.

In the ureteral stent according to the aspect, a hue of the first color may be different from a hue of the second color. Since the hues of the target curved part and the extension part are different from each other, the user can easily check the boundary position between the target curved part and the extension part when placing the ureteral stent using a cystoscope. Thus, when using the cystoscope, the user can place the ureteral stent in the body in the state in which the target curved part is appropriately disposed in the kidney or the bladder.

In the ureteral stent according to the aspect, a brightness of the second color may be greater than a brightness of the first color. Since the brightness of the extension part is higher than the brightness of the target curved part, when placing the ureteral stent in the body using the cystoscope, the user can easily check the extension part even when there is hematuria present in the urinary tract. Thus, when using the cystoscope, the user can place the ureteral stent in the body in the state in which the extension part is appropriately disposed in the ureter.

In the ureteral stent according to the aspect, the first proportion may be different from the second proportion. In this case, by causing the ratios of each of the first material and the second material to be different from each other, the ureteral stent can realize the difference in the X-ray transmittances of each of the target curved part and the extension part.

In the ureteral stent according to the aspect, the first proportion may be greater than the second proportion. In this case, the ureteral stent can cause the visibility of the target curved part under the X-ray radioscopy to be favorable.

In the ureteral stent according to the aspect, the first proportion may be greater than the second proportion by 20 wt.% or more. In this case, the ureteral stent can cause the visibility of the target curved part under the X-ray radioscopy to be even more favorable.

In the ureteral stent according to the aspect, the first material may be tungsten. By using tungsten, which is chemically stable, as the first material, the safety of the ureteral stent can be improved.

In the ureteral stent according to the aspect, the first proportion may be 50 wt.% or more. In this case, the visibility of the target curved part under the X-ray radioscopy can be made favorable.

In the ureteral stent according to the aspect, the second material may be barium sulfate. By using barium sulfate, which is chemically stable, as the second material, the safety of the ureteral stent can be improved.

In the ureteral stent according to the aspect, the second proportion may be from 10 wt.% or more to 30 wt.% or less. In this case, the visibility of the target curved part under the X-ray radioscopy can be made favorable.

In the ureteral stent according to the aspect, the target curved part may include a first section and a second section. The first section may be curved in a circular shape. The second section may be disposed inside a virtual circle along which the first section, and include an end part of the tubular body. By suppressing the first section and the second section from overlapping each other, it is possible to reduce the occurrence of metal artifacts when imaging by a CT scan using X-rays. Further, since the end portion of the tubular body is suppressed from protruding further to the outside than the first section, it is possible to prevent the end portion from becoming caught up inside the body.

In the ureteral stent according to the aspect, the target curved part may be curved in a spiral shape. A third section of the target curved part may be adjacent to the extension part. A fourth section of the target curved part may be adjacent to the end part of the tubular body. The third section and the fourth section may overlap each other in an extending direction of a spiral center. The first material may be contained in section of the target curved part excluding the third section, and the second material may be contained in the third section. By suppressing the sections of the target curved part containing the first material from overlapping each other, it is possible to reduce the occurrence of metal artifacts when imaging by the CT scan using X-rays.

In the ureteral stent according to an aspect, the target curved part may be curved in a spiral shape. A third section of the target curved part may be adjacent to the extension part. A fourth section of the target curved part may be adjacent to the end part of the tubular body. The third section and the fourth section may overlap each other in an extending direction of a spiral center. An X-ray transmittance of the third section may be lower than an X-ray transmittance of the extension part and of the fourth section. By suppressing the sections having the lower X-ray transmittance from overlapping each other, it is possible to reduce the occurrence of metal artifacts when imaging by the CT scan using X-rays.

In the ureteral stent according to an aspect, an X-ray transmittance of an adjacent part of the target curved part that is adjacent to the extension part may be lower than an X-ray transmittance of the extension part. In this case, the user can easily check the boundary position between the target curved part and the extension part under the X-ray radioscopy.

### Brief description of the drawings

Fig. 1 is a view showing a ureteral stent 1A.
Fig. 2 is an enlarged view of a first curved part 31.
Fig. 3 is an enlarged view of a second curved part 32.
Fig. 4 is a view showing a state in which the ureteral stent 1A is placed in the body.
Fig. 5 is a view showing a ureteral stent 1B.
Fig. 6 is a table showing compositions of samples 1 to 10.
Fig. 7 is a table showing evaluation results under X-ray radioscopy.
Fig. 8 is a table showing imaging results under X-ray radioscopy.
Fig. 9 is a view showing a ureteral stent 1C.
Fig. 10 is a view showing imaging results by a CT scan using X-rays.
Fig. 11 is a view showing a ureteral stent 1D.
Fig. 12 is a view showing modified examples of the ureteral stents 1A and 1C.

### Description of Embodiments

Hereinafter, an embodiment of a ureteral stent 1 according to the present invention will be described with reference to the drawings. The referenced drawings are used to describe technological features that can be adopted by the present invention. Configurations and the like of devices described herein are not intended to limit the present invention, and are simply explanatory examples.

The ureteral stent 1 is used subsequent to treatment for urinary calculosis, urological tumors, or the like, is used for post-operation drainage following an operation for prostatic hypertrophy, or for drainage in urinary tract infections, and the like. The ureteral stent 1 is placed in the body and used in a state of being disposed between the kidney and the bladder. The ureteral stent 1 fulfils roles of securing a passage for urine, facilitating the discharge of calculi from the body, reducing pain when discharging the calculi, enlarging the ureter before a procedure, and the like.

In the related art, as a method for placing the ureteral stent 1 in the body, a method using a cystoscope is used. Specifically, this is as follows. First, the cystoscope is inserted from the urethral meatus, and a position of a connection opening between the ureter and the bladder (the ureteral opening) is checked. In this state, a guide wire is inserted from the ureteral opening into the ureter. Next, the ureteral stent 1 is inserted along the guide wire, and the ureteral stent 1 is placed inside the body in a state of being disposed between the kidney and the bladder. Note that, in order to check that the one end portion of the ureteral stent 1 is disposed in the kidney and the other end portion of the ureteral stent 1 is disposed in the bladder, a contrast medium is sometimes used in conjunction with the cystoscope.

When the ureteral stent 1 is disposed at a position biased toward the kidney, it is necessary to extract the ureteral stent 1 under anesthesia. Further, when the ureteral stent 1 is placed at a position biased toward the bladder, this may lead to increased urinary frequency or pain when urinating. In contrast, instead of the method using the cystoscope of the related art, the ureteral stent 1 can be placed in the body while checking the position thereof under X-ray radioscopy. In this way, a user performing treatment on a patient using the ureteral stent 1 can place the ureteral stent 1 in the appropriate position inside the body.

### Ureteral stent 1A

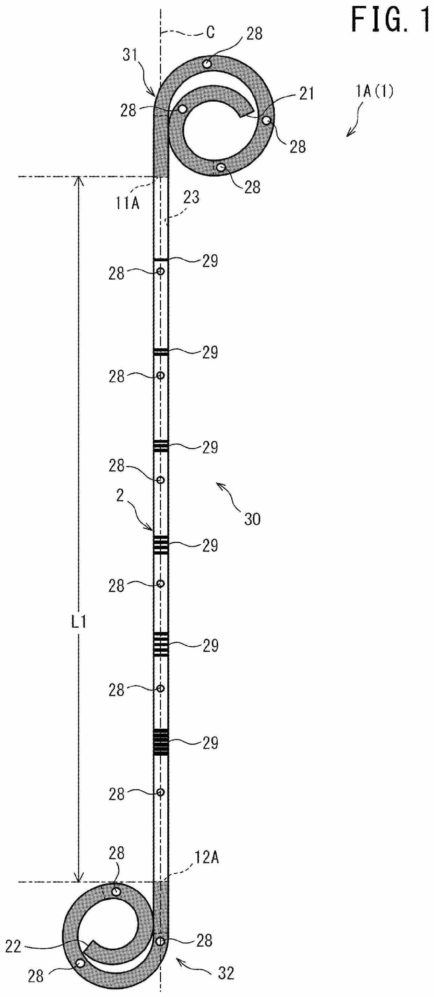

A ureteral stent 1A that is an embodiment of the ureteral stent 1 will be described with reference to Fig. 1 to Fig. 3. The ureteral stent 1A has a shape in which the end parts of a tubular body 2 are curved. The end part on one side of the tubular body 2 will be referred to as a "one end part 21", and the end part on the other side will be referred to as "another end part 22". The tubular body 2 has a cylindrical shape, and extends between the one end part 21 and the other end part 22. A cross-sectional shape of the tubular body 2 is circular. The diameter of the tubular body 2 is 2 mm, for example. The tubular body 2 includes a lumen 23. The lumen 23 extends between the one end part 21 and the other end part 22. A cross-sectional shape of the lumen 23 is circular. The diameter of the lumen 23 is 1.3 mm, for example. A direction extending along the center of the tubular body 2 will be referred to as an "extending direction".

A material of the tubular body 2 is urethane. A plurality of side holes 28 are provided in the tubular body 2. Each of the side holes 28 penetrates to the lumen 23. The plurality of side holes 28 are aligned at equal intervals in the extending direction.

The tubular body 2 includes an extension part 30, a first curved part 31, and a second curved part 32. The first curved part 31 is positioned in the vicinity of the one end part 21, and the second curved part 32 is positioned in the vicinity of the other end part 22. The extension part 30 is interposed between the first curved part 31 and the second curved part 32. The extension part 30 extends from an end portion of the first curved part 31 on the other side of the one end part 21 to an end portion of the second curved part 32 on the other side from the other end part 22. A virtual straight line extending along the extension part 30 will be referred to as a "virtual line C". A boundary between the extension part 30 and the first curved part 31 will be referred to as a "boundary 11A". A boundary between the extension part 30 and the second curved part 32 will be referred to as a "boundary 12A". A length in the extending direction between the boundaries 11A and 12A is denoted by L1. The length L1 is 300 mm, for example.

The extension part 30 is disposed in the ureter in the state in which the ureteral stent 1A is placed in the body. A plurality of markers 29 are provided on the extension part 30. Each of the plurality of markers 29 includes one or more lines extending around the surface of the tubular body 2. The plurality of markers 29 are disposed at positions that are integer multiples of a predetermined unit distance from the boundary 11A. The number of lines included in each of the plurality of markers 29 increases the greater the distance from the boundary 11A.

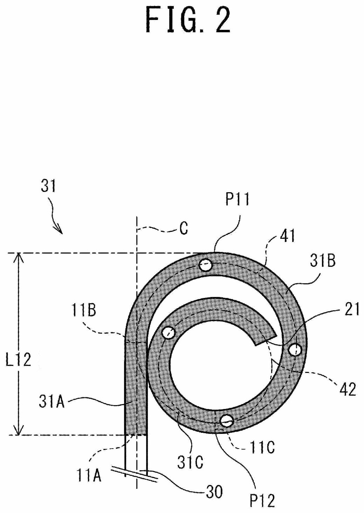

The first curved part 31 is disposed in the kidney in the state in which the ureteral stent 1A is placed in the body. As shown in Fig. 2, the first curved part 31 includes a connecting section 31A, a first section 31B, and a second section 31C. The connecting section 31A extends in a straight line along the virtual line C, from the boundary 11A toward the opposite side from the extension part 30. The first section 31B is connected to the end portion, of the connecting section 31A, on the opposite side from the boundary 11A. The second section 31C is connected to the end portion, of the first section 31B, on the opposite side from the connecting section 31A. The first section 31B and the second section 31C respectively curve in a circular shape. The first section 31B extends in the circular shape along a hypothetical virtual circle 41 that is tangential to the virtual line C passing through the connecting section 31A. Of the first curved part 31, a position separated farthest from the extension part 30 in the direction along the virtual line C will be referred to as a maximum position P11. Of the first curved part 31, a position facing the maximum position P11 with the virtual circle 41 interposed therebetween will be referred to as a maximum position P12. The second section 31C extends in the circular shape along a hypothetical virtual circle 42 that is tangential to the virtual circle 41 at the maximum position P12 and is positioned inside the virtual circle 41. The diameter of the virtual circle 42 is smaller than the diameter of the virtual circle 41. Since the virtual circle 42 is disposed inside the virtual circle 41, the second section 31C is also disposed inside the virtual circle 41. The one end part 21 of the tubular body 2 is disposed inside the first section 31B.

The extension part 30, the connecting section 31A, the first section 31B, and the second section 31C are disposed on a common virtual plane. A direction orthogonal to the virtual plane is defined as an orthogonal direction. The first section 31B and the second section 31C do not overlap each other in the orthogonal direction.

A boundary between the connecting section 31A and the first section 31B will be referred to as a "boundary 11B". A boundary between the first section 31B and the second section 31C will be referred to as a "boundary 11C". Of the second section 31C, the end portion on the opposite side from the boundary 11C is the one end part 21 of the tubular body 2. A length of the first curved part 31 is denoted by L11. The length L11 is 70 mm, for example. A length between the boundary 11A and the maximum position P11 in the direction along the virtual line C is denoted by L12. The length L12 is 17 mm, for example. The radius of curvature of the first section 31B corresponds to the radius of the virtual circle 41, and is 8 mm, for example. The radius of curvature of the second section 31C corresponds to the radius of the virtual circle 42, and is 5 mm, for example.

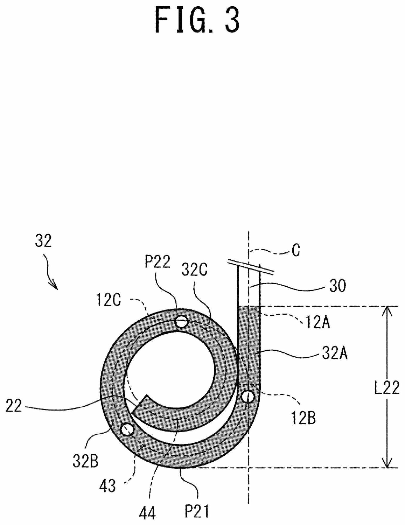

The second curved part 32 is disposed in the bladder in the state in which the ureteral stent 1A is placed in the body. As shown in Fig. 3, the second curved part 32 includes a connecting section 32A, a first section 32B, and a second section 32C. The connecting section 32A extends in a straight line along the virtual line C, from the boundary 12A toward the opposite side from the extension part 30. The first section 32B is connected to the end portion, of the connecting section 32A, on the opposite side from the boundary 12A. The second section 32C is connected to the end portion, of the first section 32B, on the opposite side from the connecting section 32A. The first section 32B and the second section 32C respectively curve in a circular shape. The first section 32B extends in the circular shape along a hypothetical virtual circle 43 that is tangential to the virtual line C passing through the connecting section 32A. Of the second curved part 32, a position separated farthest from the extension part 30 in the direction along the virtual line C will be referred to as a maximum position P21. Of the second curved part 32, a position facing the maximum position P21 with the virtual circle 43 interposed therebetween will be referred to as a maximum position P22. The second section 32C extends in the circular shape along a hypothetical virtual circle 44 that is tangential to the virtual circle 43 at the maximum position P22 and is positioned inside the virtual circle 43. The diameter of the virtual circle 44 is smaller than the diameter of the virtual circle 43. Since the virtual circle 44 is disposed inside the virtual circle 43, the second section 32C is also disposed inside the virtual circle 43. The other end part 22 of the tubular body 2 is disposed inside the first section 32B.

The extension part 30, the connecting section 32A, the first section 32B, and the second section 32C are disposed on a common virtual plane. The virtual plane coincides with the virtual plane on which the extension part 30, the connecting section 31A, the first section 31B, and the second section 31C are disposed. The first section 32B and the second section 32C do not overlap each other in the orthogonal direction orthogonal to the virtual plane.

A boundary between the connecting section 32A and the first section 32B will be referred to as a "boundary 12B". A boundary between the first section 32B and the second section 32C will be referred to as a "boundary 12C". Of the second section 32C, the end portion on the opposite side from the boundary 12C is the other end part 22 of the tubular body 2. A length of the second curved part 32 is denoted by L21. The length L21 is 50 mm, for example. A length between the boundary 12A and the maximum position P21 in the direction along the virtual line C is denoted by L22. The length L22 is 13 mm, for example. The radius of curvature of the first section 32B corresponds to the radius of the virtual circle 43, and is 6 mm, for example. The radius of curvature of the second section 32C corresponds to the radius of the virtual circle 44, and is 3 mm, for example.

The diameter of the virtual circle 43 is smaller than the diameter of the virtual circle 41 (refer to Fig. 2). The diameter of the virtual circle 44 is smaller than the diameter of the virtual circle 42 (refer to Fig. 2). As shown in Fig. 1, the first curved part 31 and the second curved part 32 are disposed on different sides with respect to the virtual line C.

First material, first proportion, second material, second proportion

An X-ray opaque material is contained in each of the extension part 30, the first curved part 31, and the second curved part 32. X-ray opaque indicates a characteristic in that a component in a substance blocks the transmission of X-rays to a certain extent. When the X-ray opaque material is contained in the extension part 30, the first curved part 31, and the second curved part 32, concentration differences with other sections occur when the ureteral stent 1A is irradiated with and examined under X-rays, and thus, the extension part 30, the first curved part 31, and the second curved part 32 can be visualized. The X-ray opaque material contained in the first curved part 31 and the second curved part 32 is the same.

Hereinafter, the X-ray opaque material contained in the first curved part 31 and the second curved part 32 will be referred to as a "first material". A weight ratio of the first material with respect to the urethane that is the material of the tubular body 2 will be referred to as a "first proportion". The X-ray opaque material contained in the extension part 30 will be referred to as a "second material". A weight ratio of the second material with respect to the urethane that is the material of the tubular body 2 will be referred to as a "second proportion". The first material, first proportion, second material, and second proportion can be changed as appropriate, as long as the X-ray transmittance of the extension part 30 differs from the X-ray transmittance of the first curved part 31 and the second curved part 32.

A specific example of the first material, the first proportion, the second material, and the second proportion is as follows. The first curved part 31 and the second curved part 32 contain 50 wt.% (first proportion) of tungsten (first material), which is an X-ray opaque material. The extension part 30 contains 10 wt.% or more to 30 wt.% or less (second proportion) of barium sulfate (second material), which is an X-ray opaque material. In this way, the first material and the second material differ from each other, and the first proportion and the second proportion differ from each other. Further, the first proportion is greater than the second proportion. More specifically, the first proportion is greater by 20 wt.% or more than the second proportion. In this way, the X-ray transmittance of the first curved part 31 and the second curved part 32 is smaller than the X-ray transmittance of the extension part 30.

### First color, second color

The color of the first curved part 31 and the second curved part 32 is the same. Hereinafter, the color of the first curved part 31 and the second curved part 32 will be referred to as a "first color", and the color of the extension part 30 will be referred to as a "second color". The first color and the second color can be changed as appropriate, as long as they satisfy conditions that they have different hues, and that the brightness of the second color is higher than the brightness of the first color.

A specific example of the first color and the second color is as follows. For example, the first color is black, and the second color is green. The hue of the first color is approximately 0° and the hue of the second color is approximately 174°. The brightness of the first color is approximately 0%, and the brightness of the second color is approximately 79%. The saturation of the first color is approximately 0%, and the saturation of the second color is approximately 84%.

Note that the first color of the first curved part 31 and the second curved part 32, and the second color of the extension part 30 may be imparted by applying a pigment or the like to a surface of the tubular body 2. Further, the first color of the first curved part 31 and the second curved part 32, and the second color of the extension part 30 may be imparted by the addition of the X-ray opaque material to each thereof (to the first material or the second material).

### Mode of use of ureteral stent 1A

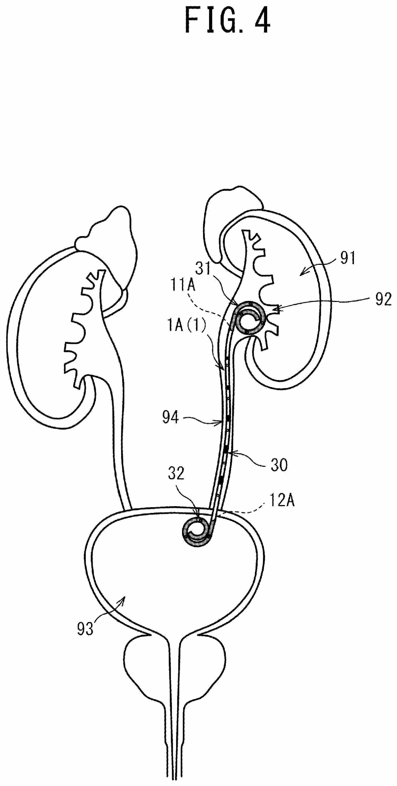

The user performing treatment on the patient using the ureteral stent 1A inserts the ureteral stent 1A into the body from the urethral meatus. The user adjusts the position of the ureteral stent 1A inside the body while checking the positions of the first curved part 31, the second curved part 32, and the extension part 30 under the X-ray radioscopy. As shown in Fig. 4, subsequent to checking that the first curved part 31 is disposed in the vicinity of the exit of a kidney 91, more specifically, at a renal pelvis 92, and that the second curved part 32 is disposed in a bladder 93, the user ends the manipulation.

### Operations and effects of ureteral stent 1A

In the ureteral stent 1A, the X-ray transmittance of the extension part 30 differs from the X-ray transmittances of each of the first curved part 31 and the second curved part 32 . Thus, under the X-ray radioscopy, the user can more easily check each of the boundary 11A between the first curved part 31 and the extension part 30, and the boundary 12A between the second curved part 32 and the extension part 30, using the concentration differences. As a result, under the X-ray radioscopy, the user can place the ureteral stent 1A in the body in the state in which the first curved part 31 is appropriately disposed in the kidney 91 and the second curved part 32 is appropriately disposed in a bladder 93.

In the ureteral stent 1A, the first material that is the material of the first curved part 31 and the second curved part 32, and the second material that is the material of the extension part 30 are different from each other. In this case, the ureteral stent 1A can easily realize the difference between the X-ray transmittance of each of the first curved part 31 and the second curved part 32, and that of the extension part 30 by causing the first material and the second material to be different.

In the ureteral stent 1A, the first proportion that is the ratio by weight of the material of the first curved part 31 and the second curved part 32 (first material), and the second proportion that is the ratio by weight of the material of the extension part 30 (second material) are different from each other. In this case, the ureteral stent 1A can easily realize the difference between the X-ray transmittance of each of the first curved part 31 and the second curved part 32, and that of the extension part 30 by causing the ratios of the first material and the second material to be different. Further, by causing the first proportion to be greater by 20 wt.% or more than the second proportion, it is possible to increase the concentration differences under the X-ray radioscopy, and it is thus possible to enhance the visibility of the boundary 11A between the first curved part 31 and the extension part 30, and the boundary 12A between the second curved part 32 and the extension part 30. Thus, the user can easily check whether the first curved part 31 is disposed in the kidney 91, and whether the second curved part 32 is disposed in the bladder 93.

Tungsten is used as the material (first material) of the first curved part 31 and the second curved part 32. Tungsten has a high specific gravity, and a high X-ray shielding capability. Further, tungsten is known for having a high melting point and being chemically stable. Thus, by using tungsten as the first material, the safety of the ureteral stent 1A can be improved.

Barium sulfate is used as the material (second material) of the extension part 30. Barium sulfate has a high rate of absorption of X-rays. Further, barium sulfate is known for being water insoluble and harmless to the human body. Thus, by using barium sulfate as the second material, the safety of the ureteral stent 1A can be improved.

Further, by using tungsten as the first material and barium sulfate as the second material, even when the strength of the X-rays irradiated for the radioscopy is lowered, the ureteral stent 1A can be easily visually checked. Thus, it is possible to reduce an amount of exposure of the patient or the user.

The one end part 21 of the tubular body 2 is disposed inside the first section 31B, and the other end part 22 of the tubular body 2 is disposed inside the first section 32B. Thus, by suppressing the one end part 21 and the other end part 22 from protruding further to the outside than the first sections 31B and 32B, the ureteral stent 1A can prevent the one end part 21 and the other end part 22 from becoming caught up inside the body.

The user may sometimes place the ureteral stent 1A in the body using the cystoscope. Here, in the ureteral stent 1A, the hue of the first color that is the color of the first curved part 31 and the second curved part 32, and the hue of the second color that is the color of the extension part 30 are different from each other. Thus, when placing the ureteral stent 1A in the body using the cystoscope, the user can easily check the boundary 11A between the first curved part 31 and the extension part 30 and the boundary 12A between the second curved part 32 and the extension part 30 using the differences in hue of each of the colors. As a result, when the cystoscope is used, the user can place the ureteral stent 1A in the body in the state in which the first curved part 31 is appropriately disposed in the kidney 91 and the second curved part 32 is appropriately disposed in the bladder 93.

In the case in which the user places the ureteral stent 1A in the body using the cystoscope, when there is hematuria present in the urinary tract, for example, visibility of the extension part 30 disposed in a ureter 94 may deteriorate. Here, in the ureteral stent 1A, the second color that is the color of the extension part 30 has a higher brightness than the first color that is the color of the first curved part 31 and the second curved part 32. Thus, even when hematuria is present in the urinary tract, the user can easily check the extension part 30. Thus, even when the cystoscope is used, the user can place the ureteral stent 1A in the body in the state in which the extension part 30 is appropriately disposed in the ureter 94.

### Ureteral stent 1B

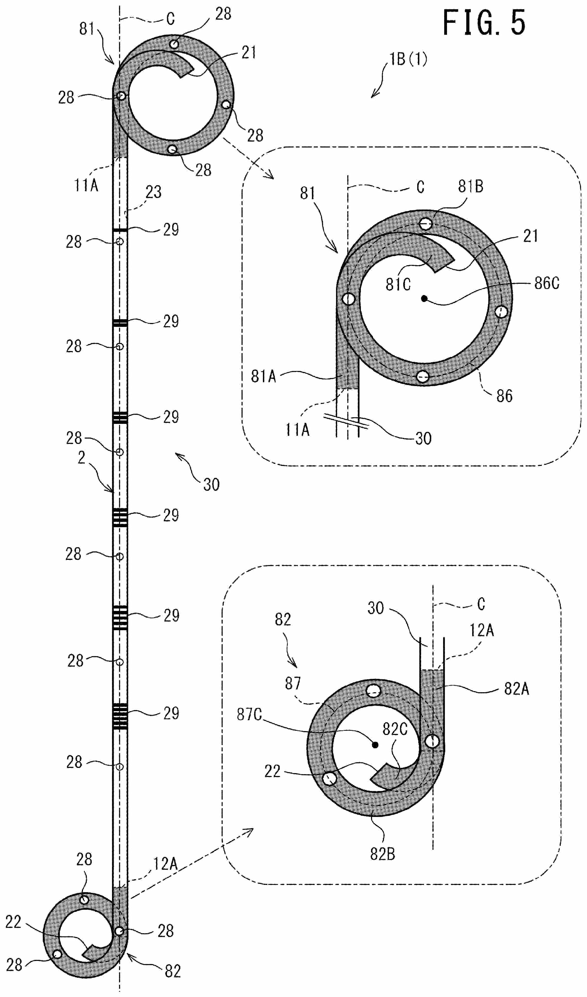

A ureteral stent 1B that is an embodiment of the ureteral stent 1 will be described with reference to Fig. 5. The tubular body 2 of the ureteral stent 1B includes the extension part 30, a first curved part 81, and a second curved part 82. The shape of the extension part 30 is the same as that of the extension part 30 of the ureteral stent 1A. The shapes of the first curved part 81 and the second curved part 82 differ from those of the first curved part 31 and the second curved part 32 of the ureteral stent 1A (refer to Fig. 1). The boundary 11A is the boundary between the extension part 30 and the first curved part 81. The boundary 12A is the boundary between the extension part 30 and the second curved part 82.

The first curved part 81 is disposed in the kidney in the state in which the ureteral stent 1B is placed in the body. The first curved part 81 includes a connecting section 81A and a spiral section 81B. The connecting section 81A extends in a straight line along the virtual line C, from the boundary 11A toward the opposite side from the extension part 30. The spiral section 81B is connected to the end portion, of the connecting section 81A, on the opposite side from the boundary 11A. The spiral section 81B curves in a spiral shape. A rotational center of the spiral section 81B will be referred to as a "spiral center 86C". The spiral center 86C extends in a straight line shape. When viewed from one side in the extending direction of the spiral center 86C, the spiral section 81B extends along a hypothetical virtual circle 86 centered on the spiral center 86C. Parts of the spiral section 81B overlap each other in the extending direction of the spiral center 86C. The end portion of the spiral section 81B on the opposite side from the connecting section 81A is the one end part 21. In the vicinity of the one end part 21, the spiral section 81B includes a protrusion 81C that protrudes toward the inside of the virtual circle 86.

The second curved part 82 is disposed in the bladder in the state in which the ureteral stent 1B is placed in the body. The second curved part 82 includes a connecting section 82A and a spiral section 82B. The connecting section 82A extends in a straight line along the virtual line C, from the boundary 12A toward the opposite side from the extension part 30. The spiral section 82B is connected to the end portion, of the connecting section 82A, on the opposite side from the boundary 12A. The spiral section 82B curves in a spiral shape. A rotational center of the spiral section 82B will be referred to as a "spiral center 87C". The spiral center 87C extends in a straight line shape. When viewed from one side in the extending direction of the spiral center 87C, the spiral section 82B extends along a hypothetical virtual circle 87 centered on the spiral center 87C. Parts of the spiral section 82B overlap each other in the extending direction of the spiral center 87C. The end portion of the spiral section 82B on the opposite side from the connecting section 82A is the other end part 22. In the vicinity of the other end part 22, the spiral section 82B includes a protrusion 82C that protrudes toward the inside of the virtual circle 87.

The diameter of the virtual circle 87 is smaller than the diameter of the virtual circle 86. The first curved part 81 and the second curved part 82 are disposed on different sides with respect to the virtual line C.

The X-ray opaque first material of the first proportion is contained in the first curved part 81 and the second curved part 82. The color of the first curved part 81 and the second curved part 82 is the first color. The X-ray opaque second material of the second proportion is contained in the extension part 30. The color of the extension part 30 is the second color. The first material is tungsten, for example. The first proportion is 50 wt.%, for example. The first color is black, for example. The second material is barium sulfate, for example. The second proportion is 10 wt.% or more to 30 wt.% or less, for example. The second color is green, for example.

### Operations and effects of ureteral stent 1B

The ureteral stent 1B achieves the same effects as those of the ureteral stent 1A. Further, in the ureteral stent 1B, the one end part 21 of the tubular body 2 is disposed inside the spiral section 81B, and the other end part 22 of the tubular body 2 is disposed inside the spiral section 82B. Thus, by suppressing the one end part 21 and the other end part 22 from protruding further to the outside than the virtual circles 86 and 87, the ureteral stent 1B can prevent the one end part 21 and the other end part 22 from becoming caught up inside the body.

### Evaluation 1

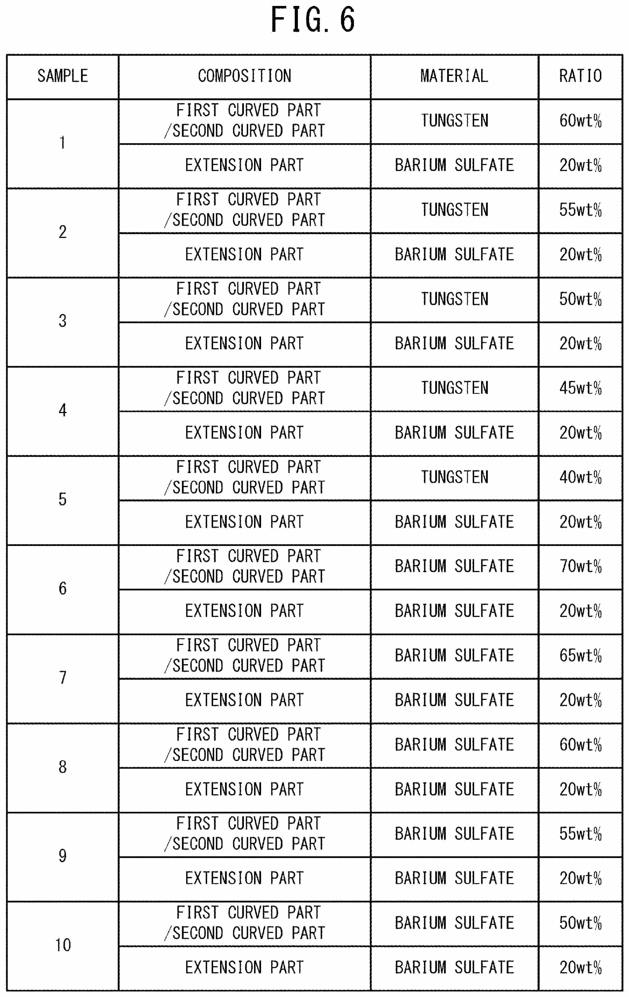

An evaluation was performed with respect to the visibility of the ureteral stent 1B under X-ray radioscopy. Samples 1 to 10 shown in Fig. 6 were prepared. Further, for the purpose of comparison with the samples 1 to 10, a push catheter TG1126022 (comparative sample 1) manufactured by Goodman Inc., an Inlay Optima 788622 (comparative sample 2) manufactured by Bird (Becton, Dickinson and Company), and a push catheter (with the inlay optima, comparative example 3) manufactured by Bird (Becton, Dickinson and Company) were prepared. The visibility of these samples under X-ray radioscopy was evaluated in a state in which the sample was fixed to a phantom (material: acrylic resin, thickness: 20 cm). The evaluation was performed both in a state in which the guide wire was inserted through the lumen 23 of the tubular body 2, and in a state in which the guide wire was not inserted. The following three evaluation criteria were set and a three-stage evaluation (Good, Average, Bad) was carried out for each.
(1) Can the first curved part 81 and the second curved part 82 be clearly recognized?
(2) Can the extension part 30 be visualized?
(3) In the state in which the guide wire is inserted through the lumen 23 of the tubular body 2, can the differences between the shapes of the first curved part 81, the second curved part 82, and the extension part 30 be distinguished?

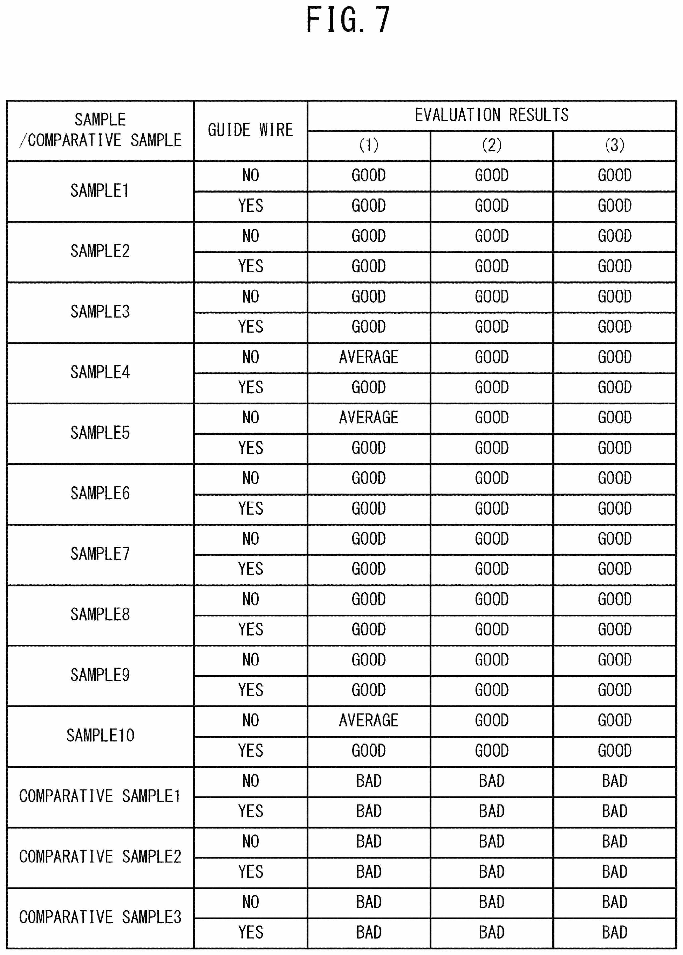

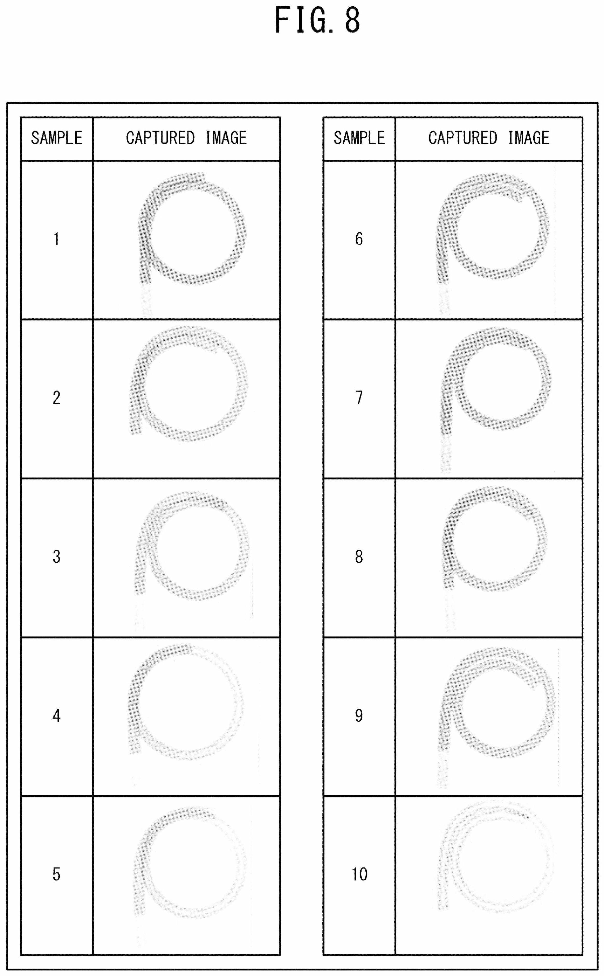

The evaluation results are shown in Fig. 7. Further, results of images obtained by imaging the samples in the state in which the guide wire is not inserted using an X-ray nondestructive testing device (µRay 6800, manufactured by MATSUSADA PRECISION Inc.) are shown in Fig. 8. Note that, since variations occur in the dimensions of the first curved part 81 and the second curved part 82 at the time of manufacturing the samples 1 to 10, shapes shown by the imaging results in Fig. 8 vary for each of the samples 1 to 10.

From the results shown in Fig. 7 and Fig. 8, it was understood that the first curved part 81, the second curved part 82, and the extension part 30 can be visualized under X-ray radioscopy, by using tungsten or barium sulfate as the material (first material) of the first curved part 81 and the second curved part 82, and barium sulfate as the material (second material) of the extension part 30. Further, it was understood that the boundaries 11A and 12A between the first curved part 81 and the second curved part 82, and the extension part 30 can be favorably visualized under the X-ray radioscopy by causing the ratio by weight (first proportion) of the X-ray opaque material contained in the first curved part 81 and the second curved part 82 to be greater by 20 wt.% or more than the ratio by weight (second proportion) of the X-ray opaque material contained in the extension part 30.

Further, when the first material contained in the first curved part 81 and the second curved part 82 was tungsten, it was understood that the first curved part 81 and the second curved part 82 can be even more favorably visualized under the X-ray radioscopy by causing the first proportion to be 50 wt.% or more. In addition, when barium sulfate was used as the first material contained in the first curved part 81 and the second curved part 82, it was understood that the first curved part 81 and the second curved part 82 can be favorably visualized under the X-ray radioscopy by causing the first proportion to be 55 wt.% or more. Furthermore, when barium sulfate was used as the second material contained in the extension part 30, it was understood that the extension part 30 can be favorably visualized under the X-ray radioscopy by causing the second proportion to be at least 20 wt.%. Furthermore, even when the second proportion is 10 wt.% or more to 30 wt.% or less, it was verified that the same results as those in Fig. 7 and Fig. 8 can be obtained. Thus, when barium sulfate was used as the second material contained in the extension part 30, it was understood that the extension part 30 can be favorably visualized under the X-ray radioscopy by causing the second proportion to be 10 wt.% or more to 30 wt.% or less.

### Ureteral stent 1C

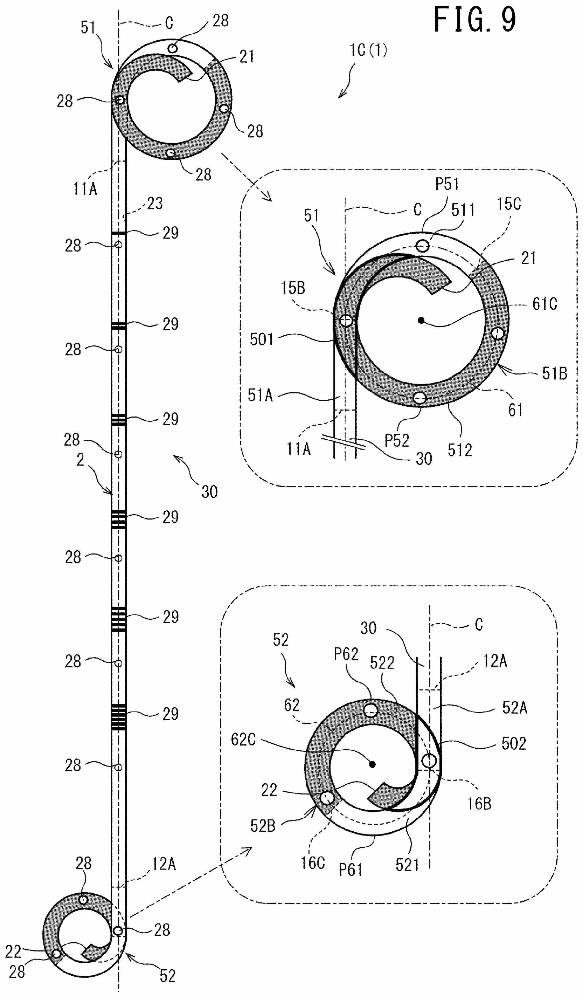

A ureteral stent 1C that is an embodiment of the ureteral stent 1 will be described with reference to Fig. 9. The tubular body 2 of the ureteral stent 1C includes the extension part 30, a first curved part 51, and a second curved part 52. The shape of the extension part 30 is the same as that of the extension part 30 of the ureteral stents 1A (refer to Fig. 1) and 1B (refer to Fig. 5). The shapes of the first curved part 51 and the second curved part 52 differ from those of the first curved part 31 and the second curved part 32 of the ureteral stent 1A (refer to Fig. 1) and are the same as those of the first curved part 81 and the second curved part 82 of the ureteral stent 1B (refer to Fig. 5). The boundary 11A is the boundary between the extension part 30 and the first curved part 51. The boundary 12A is the boundary between the extension part 30 and the second curved part 52.

The first curved part 51 is disposed in the kidney in the state in which the ureteral stent 1C is placed in the body. The first curved part 51 includes a connecting section 51A and a spiral section 51B. The connecting section 51A extends in a straight line along the virtual line C, from the boundary 11A toward the opposite side from the extension part 30. The spiral section 51B curves in a spiral shape. A rotational center of the spiral section 51B will be referred to as a "spiral center 61C". The spiral center 61C extends in a straight line shape. When viewed from one side in the extending direction of the spiral center 61C, the spiral section 51B extends along a hypothetical virtual circle 61 centered on the spiral center 61C.

The spiral section 51B includes a third section 511 and a fourth section 512. The third section 511 is connected to the end portion, of the connecting section 51A, on the opposite side from the boundary 11A. The fourth section 512 is connected to the end portion, of the third section 511, on the opposite side from the connecting section 51A. The third section 511 and the fourth section 512 have an overlapping portion 501 at which they overlap in the extending direction of the spiral center 61C.

Of the first curved part 51, a position separated farthest from the extension part 30 in the direction along the virtual line C will be referred to as a maximum position P51. Of the first curved part 51, a position facing the maximum position P51 with the spiral center 61C interposed therebetween will be referred to as a maximum position P52. A boundary between the connecting section 51A and the third section 511 will be referred to as a "boundary 15B". A boundary between the third section 511 and the fourth section 512 will be referred to as a "boundary 15C". The boundary 15C is positioned closer to the maximum position P51 than an intermediate position between the maximum positions P51 and P52. The end portion of the fourth section 512 on the opposite side from the boundary 15C is the one end part 21.

The second curved part 52 is disposed in the bladder in the state in which the ureteral stent 1C is placed in the body. The second curved part 52 includes a connecting section 52A and a spiral section 52B. The connecting section 52A extends in a straight line along the virtual line C, from the boundary 12A toward the opposite side from the extension part 30. The spiral section 52B curves in a spiral shape. A rotational center of the spiral section 52B will be referred to as a "spiral center 62C". The spiral center 62C extends in a straight line shape. When viewed from one side in the extending direction of the spiral center 62C, the spiral section 52B extends along a hypothetical virtual circle 62 centered on the spiral center 62C.

The spiral section 52B includes a third section 521 and a fourth section 522. The third section 521 is connected to the end portion, of the connecting section 52A, on the opposite side from the boundary 12A. The fourth section 522 is connected to the end portion, of the third section 521, on the opposite side from the connecting section 52A. The third section 521 and the fourth section 522 have an overlapping portion 502 at which they overlap in the extending direction of the spiral center 62C.

Of the second curved part 52, a position separated farthest from the extension part 30 in the direction along the virtual line C will be referred to as a maximum position P61. Of the second curved part 52, a position facing the maximum position P21 with the spiral center 62C interposed therebetween will be referred to as a maximum position P62. A boundary between the connecting section 52A and the third section 521 will be referred to as a "boundary 16B". A boundary between the third section 521 and the fourth section 522 will be referred to as a "boundary 16C". The boundary 16C is positioned closer to the maximum position P61 than an intermediate position between the maximum positions P61 and P62. The end portion of the fourth section 522 on the opposite side from the boundary 16C is the other end part 22.

The diameter of the virtual circle 62 is smaller than the diameter of the virtual circle 61. The first curved part 51 and the second curved part 52 are disposed on different sides with respect to the virtual line C.

The X-ray opaque material contained in the connecting section 51A and the third section 511 of the first curved part 51, and the X-ray opaque material contained in the connecting section 52A and the third section 521 of the second curved part 52 are both the second material that is the same material of the extension part 30, and are barium sulfate, for example. On the other hand, the X-ray opaque material contained in the fourth section 512 of the first curved part 51 and the X-ray opaque material contained in the fourth section 522 of the second curved part 52 are both the first material, and are tungsten, for example. Thus, of the first curved part 51 and the second curved part 52, the X-ray opaque materials contained at the overlapping portions 501 and 502 that overlap in the extending direction of the spiral centers 61C and 62C are different.

Further, in accordance with the contained X-ray opaque materials being different, the colors at respective portions also differ. A specific example is as follows. The color of the connecting section 51A and the third section 511 of the first curved part 51 is the second color (green, for example), and the color of the fourth section 512 of the first curved part 51 is the first color (black, for example) different from the second color. In a similar manner, the color of the connecting section 52A and the third section 521 of the second curved part 52 is the second color (green, for example), and the color of the fourth section 522 of the second curved part 52 is the first color (black, for example) different from the second color.

### Operations and effects of ureteral stent 1C

There are cases in which the ureteral stent 1C inside the body is imaged by a CT scan using X-rays. Further, in the CT scan, there is a possibility that, when sections at which X-ray transmittance is extremely low are contained in an imaging target, this may result in metal artifacts. The metal artifacts appear in a captured image as disturbances and noise around the sections at which the X-ray transmittance is extremely low. Thus, in order to accurately analyze the captured image, it is preferable to suppress the occurrence of the metal artifacts.

Here, when the first material is contained in all the sections of each of the first curved part 81 and the second curved part 82, as in the ureteral stent 1B (refer to Fig. 5), there is a possibility that the X-ray transmittance may be extremely low at the overlapping portions. This is because the X-ray transmittance of the sections including the first material is set to be smaller than the X-ray transmittance of the sections containing the second material. In other words, in the ureteral stent 1B, the sections containing the first material having the low X-ray transmittance overlap each other, and thus, the metal artifacts easily occur at the overlapping portions.

It should be noted that when the only object is to suppress the metal artifacts, other measures are conceivable, such as shortening the length of each of the first curved part 81 and the second curved part 82, forming the first curved part 81 and the second curved part 82 such that the overlapping portions are not formed, and the like. However, in these cases, it becomes difficult to hold the shape of the first curved part 81 and the second curved part 82, and there is a possibility that the ureteral stent 1B inside the body may become dislodged, for example. Here, in the ureteral stent 1C, it is possible to secure the length of each of the first curved part 51 and the second curved part 52, and form the overlapping portions 501 and 502 so as to suppress the ureteral stent 1C from becoming dislodged from inside the body, and at the same time, to suppress the occurrence of the metal artifacts.

In the ureteral stent 1C, the X-ray opaque material contained in the connecting section 51A and the third section 511 of the first curved part 51, and the X-ray opaque material contained in the connecting section 52A and the third section 521 of the second curved part 52 are both the second material. Thus, at the overlapping portions 501 and 502, the sections containing the first material do not overlap each other. Thus, when the ureteral stent 1C is imaged by the CT scan using the X-rays, it is possible to suppress the occurrence of the metal artifacts in the captured image.

### Evaluation 2

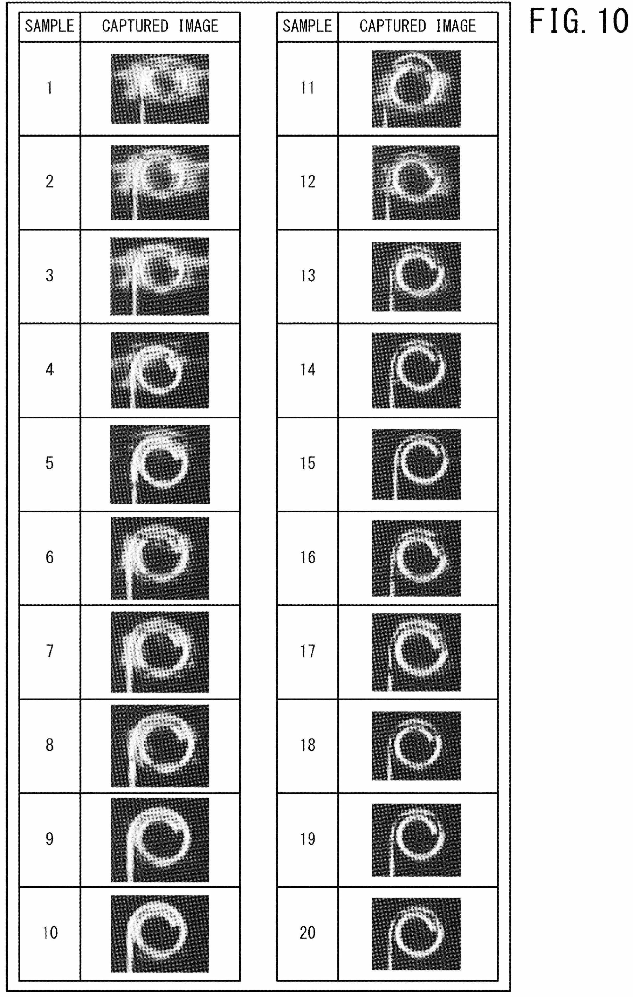

An evaluation was performed with respect to results of a CT scan using X-rays. As shown by the ureteral stent 1C, samples 11 to 20 were prepared in which the material of the extension part 30, the connecting sections 51A and 52A, and the third sections 511 and 521 was the second material, and the material of the fourth sections 512 and 522 was the first material. The samples 1 to 10 of Evaluation 1 (the ureteral stent 1B) were also used. The types and ratios of the first material and the second material in the samples 11 to 20 were the same as those for the samples 1 to 10 (refer to Fig. 6). The evaluation was performed by fixing each of the samples 1 to 20 to a phantom (Tough Water Phantom WD-4050 manufactured by Kyoto Kagaku Co. Ltd.), imaging each of the samples 1 to 20 by irradiating X-rays using a CT device, and comparing imaging results. The evaluation results are shown in Fig. 10. Note that, since variations occur in the dimensions of the first curved parts 51, 81 and the second curved parts 52, 82 at the time of manufacturing the samples 1 to 20, shapes shown by the evaluation results in Fig. 10 also vary for each of the samples 1 to 20.

From the results shown in Fig. 10, compared to the imaging results when the first material is contained in all the sections of each of the first curved part 81 and the second curved part 82 (samples 1 to 10), disturbances caused by metal artifacts were suppressed in the imaging results when the second material is contained in the connecting sections 51A and 52A, and in the third sections 511 and 521 (samples 11 to 20). Thus, it was understood that the CT scan targeting the ureteral stent 1C can be accurately executed by adopting the configuration in which the second material is contained in the connecting section 51A and the third section 511 of the first curved part 51, and in the connecting section 52A and the third section 521 of the second curved part 52, and in which the first material is contained in the fourth section 512 of the first curved part 51 and in the fourth section 522 of the second curved part 52.

### Ureteral stent 1D

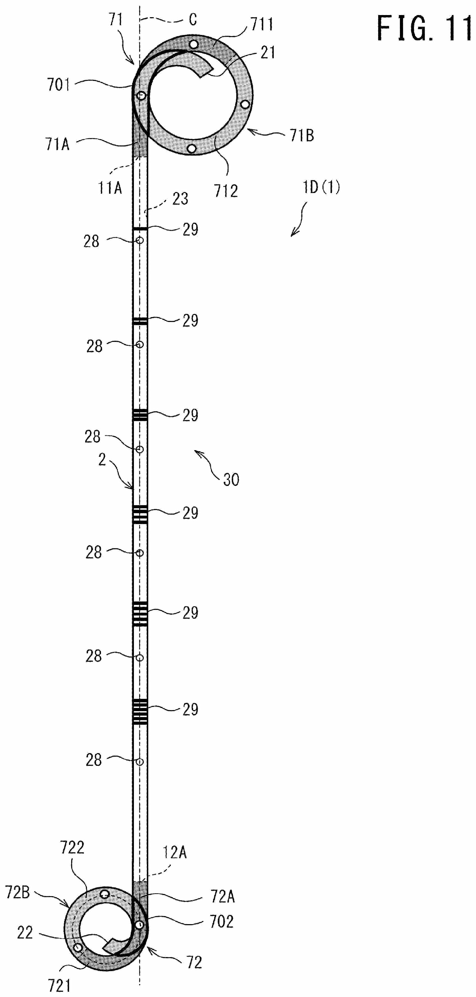

A ureteral stent 1D that is an embodiment of the ureteral stent 1 will be described with reference to Fig. 11. The tubular body 2 of the ureteral stent 1D includes the extension part 30, a first curved part 71, and a second curved part 72. The shape of the first curved part 71 is the same as that of the first curved part 81 of the ureteral stent 1B (refer to Fig. 5) and the first curved part 51 of the ureteral stent 1C (refer to Fig. 9). The shape of the second curved part 72 is the same as that of the second curved part 82 of the ureteral stent 1B (refer to Fig. 5) and the second curved part 52 of the ureteral stent 1C (refer to Fig. 9). The extension part 30 is the same as the extension part 30 of the ureteral stents 1A (refer to Fig. 1), 1B (refer to Fig. 5) and 1C (refer to Fig. 9).

The first curved part 71 includes a connecting section 71A and a spiral section 71B. The spiral section 71B includes a third section 711 and a fourth section 712. The connecting section 71A, the spiral section 71B, the third section 711, and the fourth section 712 respectively correspond to the connecting section 51A, the spiral section 51B, the third section 511, and the fourth section 512 of the first curved part 51 of the ureteral stent 1C (refer to Fig. 9). The second curved part 72 includes a connecting section 72A and a spiral section 72B. The spiral section 72B includes a third section 721 and a fourth section 722. The connecting section 72A, the spiral section 72B, the third section 721, and the fourth section 722 respectively correspond to the connecting section 52A, the spiral section 52B, the third section 521, and the fourth section 522 of the second curved part 52 of the ureteral stent 1C (refer to Fig. 9).

The X-ray opaque material and the ratio by weight thereof contained in the connecting sections 71A and 72A and in the third sections 711 and 721 are adjusted such that the X-ray transmittance of the connecting sections 71A and 72A and the third sections 711 and 721 is lower than the X-ray transmittance of the extension part 30 and the fourth sections 712 and 722. For example, the first material (tungsten, for example) is contained as the X-ray opaque material in the connecting sections 71A and 72A, the third sections 711 and 721, and the fourth sections 712 and 722, and the second material (barium sulfate, for example) is contained as the X-ray opaque material in the extension part 30. Further, the ratio by weight of the first material contained in the connecting sections 71A and 72A, and in the third sections 711 and 712 is set to be a larger value than the ratio by weight of the first material contained in the fourth sections 712 and 722. In this way, the X-ray transmittance of the connecting sections 71A and 72A and in the third sections 711 and 721 is adjusted to be lower than the X-ray transmittance of the extension part 30 and the fourth sections 712 and 722.

In addition, the hues of the color of the extension part 30, the color of the connecting sections 71A and 72A and the third sections 711 and 721, and the color of the fourth sections 712 and 722 are respectively different. Further, the brightness of each of the colors increases in the order of the brightness of the color of the connecting sections 71A and 72A and the third sections 711 and 721, the brightness of the color of the fourth sections 712 and 722, and the brightness of the color of the extension part 30.

### Operations and effects of ureteral stent 1D

It is possible to suppress the X-ray transmittance of an overlapping section 701 at which the connecting section 71A and the third section 711 of the first curved part 71 overlap the fourth section 712 from becoming extremely low. Further, it is possible to suppress the X-ray transmittance of an overlapping section 702 at which the connecting section 72A and the third section 721 of the second curved part 72 overlap the fourth section 722 from becoming extremely low. In this way, in the ureteral stent 1D, by suppressing the sections having the low X-ray transmittance from overlapping each other, it is possible to reduce the occurrence of the metal artifacts in the captured image when imaging by the CT scan.

### Modified examples

The present invention is not limited to the above-described embodiment, and various modifications are possible. Insofar as there is no particular description otherwise, the modified examples will be described using the ureteral stent 1A as an example, but can also be applied to the ureteral stents 1B, 1C, and 1D as appropriate.

The shapes of the first curved part 31 and the second curved part 32 are not limited to being circular, and may have another shape suitable for being disposed in the kidney 91 (the renal pelvis 92), or in the bladder 93. The specific example of the first material that is the X-ray opaque material contained in the first curved part 31 and the second curved part 32 and of the first proportion that is the ratio by weight thereof, and the specific example of the second material that is the X-ray opaque material contained in the extension part 30 and of the second proportion that is the ratio by weight thereof are merely examples, and may be other materials and other ratios.

Each of the first material contained in the first curved part 31 and the second curved part 32, and the second material contained in the extension part 30 may be the same X-ray opaque material. The first proportion that is the ratio by weight of the first material contained in the first curved part 31 and the second curved part 32 may be smaller than the second proportion that is the ratio by weight of the second material contained in the extension part 30. The first proportion and the second proportion may be the same as each other. The hue and brightness of the first color that is the color of the first curved part 31 and the second curved part 32 may be the same as the hue and brightness of the second color that is the color of the extension part 30. The brightness of the first color may be greater than the brightness of the second color. The X-ray transmittance of the first curved part 31 and the second curved part 32 may be higher than the X-ray transmittance of the extension part 30.

In the ureteral stent 1A, the first material may be contained in only one of the first curved part 31 and the second curved part 32, and not necessarily be contained in the other of the first curved part 31 and the second curved part 32. For example, in the ureteral stent 1A, the first material may be contained only in the first curved part 31 whose position is hard to check using a cystoscope, and not necessarily be contained in the second curved part 32.

The X-ray opaque material contained in the first curved part 31 may be different from the X-ray opaque material contained in the second curved part 32. The X-ray transmittance of the first curved part 31 and the X-ray transmittance of the second curved part 32 may be different from each other within a range over which the condition is satisfied that the X-ray transmittance of the first curved part 31 and the second curved part 32 is different from the X-ray transmittance of the extension part 30. For example, the X-ray transmittance of the extension part 30 may be higher than the X-ray transmittance of the first curved part 31, and the X-ray transmittance of the second curved part 32 may be higher than the X-ray transmittance of the extension part 30.

The first section 31B of the first curved part 31 is not limited to extending along the virtual circle 41 that is the perfect circle, and may extend along a virtual circle that has an elliptical shape, for example. The second section 31C of the first curved part 31 is not limited to extending along the virtual circle 42 that is the perfect circle, and may extend along a virtual circle that has an elliptical shape, for example. This also applies to the first section 32B and the second section 32C of the second curved part 32.

The second section 31C of the first curved part 31 may extend directly toward the inside from the boundary 11C with the first section 31B. This also applies to the second section 32C of the second curved part 32.

Instead of the first material, the second material that is the same X-ray opaque material contained in the extension part 30 may be contained in the second section 31C of the first curved part 31, and the second section 32C of the second curved part 32. The first material may be contained only in the connecting section 31A of the first curved part 31 and the connecting section 32A of the second curved part 32. In this case, the first section 31B and the second section 31C of the first curved part 31, and the first section 32B and the second section 32C of the second curved part 32 may contain the same second material as the extension part 30.

In the ureteral stent 1D, the connecting sections 71A and 72A and the third sections 711 and 721 may contain the first material (tungsten, for example), and the fourth sections 712 and 722 and the extension part 30 may contain the second material (barium sulfate, for example). In other words, the fourth section 712 of the first curved part 71 and the fourth section 722 of the second curved part 72 may contain the same second material as the X-ray opaque material contained in the extension part 30.

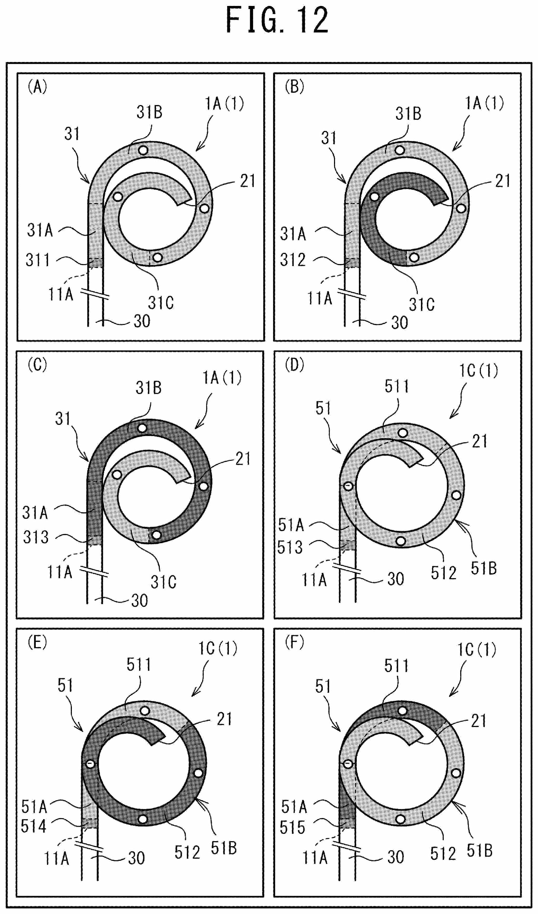

In the ureteral stent 1A, the X-ray transmittance of sections of the first curved part 31 and the second curved part 32 adjacent to the extension part 30 (hereinafter referred to as adjacent parts 311 to 313, refer to Fig. 12(A) to Fig. 12(C)) may be lower than the X-ray transmittance of the extension part 30. In a similar manner, in the ureteral stent 1C, the X-ray transmittance of sections of the first curved part 51 and the second curved part 52 adjacent to the extension part 30 (hereinafter referred to as adjacent parts 513 to 515, refer to Fig. 12(D) to Fig. 12(F)) may be lower than the X-ray transmittance of the extension part 30.

For example, as shown in Fig. 12(A) to Fig. 12(C), a portion of the connecting section 31A of the first curved part 31 including the boundary 11A with the extension part 30 corresponds to the adjacent parts 311 to 313. Here, the X-ray transmittance of the extension part 30 is denoted by x(0). The X-ray transmittance of the adjacent parts 311 to 313 is denoted by x(1). The X-ray transmittance of sections of the connecting section 31A other than the adjacent parts 311 to 313 is denoted by x(2). The X-ray transmittance of the first section 31B of the first curved part 31 is denoted by x(3). The X-ray transmittance of second section 31C of the first curved part 31 is denoted by x(4). In Fig. 12(A) to Fig. 12(C), the X-ray transmittance x(1) of the adjacent parts 311 to 313 is lower than the X-ray transmittance x(0) of the extension part 30.

In Fig. 12(A), each of the values of the X-ray transmittances x(1), x(2), x(3), and x(4) satisfy a relationship of x(1) < x(2) = x(3) = x (4). In Fig. 12(B), each of the values of the X-ray transmittances x(1), x(2), x(3), and x(4) satisfy relationships of x(1) < x(2) = x(3) > x (4), x(1) > x(4). In Fig. 12(C), each of the values of the X-ray transmittances x(1), x(2), x(3), and x(4) satisfy relationships of x(1) > x(2) = x(3) < x (4), x(1) < x(4).

As shown in Fig. 12(D) to Fig. 12(F), in the ureteral stent 1C, a portion of the connecting section 51A of the first curved part 51 including the boundary 11A with the extension part 30 corresponds to the adjacent parts 513 to 515. Here, the X-ray transmittance of the adjacent parts 513 to 515 is denoted by x(5). The X-ray transmittance of sections of the connecting section 51A other than the adjacent parts 513 to 515 is denoted by x(6). The X-ray transmittance of the third section 511 of the first curved part 51 is denoted by x(7). The X-ray transmittance of fourth section 512 of the first curved part 51 is denoted by x(8). In Fig. 12(D) to Fig. 12(F), the X-ray transmittance x(5) of the adjacent parts 513 to 515 is lower than the X-ray transmittance x(0) of the extension part 30.

In Fig. 12(D), each of the values of the X-ray transmittances x(5), x(6), x(7), and x(8) satisfy a relationship of x(5) < x(6) = x(7) = x(8). In Fig. 12(E), each of the values of the X-ray transmittances x(5), x(6), x(7), and x(8) satisfy relationships of x(5) < x(6) = x(7) > x(8), x(5) > x(8). In Fig. 12(F), each of the values of the X-ray transmittances x(5), x(6), x(7), and x(8) satisfy relationships of x(5) > x(6) = x(7) < x(8), x(5) < x(8).

As described above, by causing the X-ray transmittance of the adjacent parts 311 to 313 of the first curved part 31 and of the adjacent parts 513 to 515 of the first curved part 51 to be lower than the X-ray transmittance of the extension part 30, the visibility of the boundary 11A under X-ray radioscopy can be improved.

Note that in Figs. 12, magnitude relationships of the X-ray transmittances x(1) to x(4) and magnitude relationships of the X-ray transmittances x(5) to x(8) may be changed as necessary as long as the condition is satisfied that the X-ray transmittances x(1) and x(5) are lower than the X-ray transmittance of the extension part 30. The X-ray transmittances x(1) to x(4) and the X-ray transmittances x(5) to x(8) may be higher than the X-ray transmittance x(0) of the extension part 30, or may be lower.

## Claims

1. A ureteral stent comprising:
a tubular body extending between one end part and another end part, wherein
the tubular body includes
a first curved part configured to be disposed in a kidney,
a second curved part configured to be disposed in a bladder, and
an extension part extending between the first curved part and the second curved part,
a first material that is an X-ray opaque material is contained, in a first proportion, in at least part of a target curved part, the target curved part being at least one of the first curved part and the second curved part,
a color of at least part of the target curved part is a first color,
a second material that is an X-ray opaque material is contained, in a second proportion, in the extension part,
a color of the extension part is a second color, and
an X-ray transmittance of the target curved part is different from an X-ray transmittance of the extension part.

2. The ureteral stent according to claim 1, wherein
the first material is different from the second material.

3. The ureteral stent according to claim 1, wherein
a hue of the first color is different from a hue of the second color.

4. The ureteral stent according to claim 1, wherein
a brightness of the second color is greater than a brightness of the first color.

5. The ureteral stent according to claim 1, wherein
the first proportion is different from the second proportion.

6. The ureteral stent according to claim 5, wherein
the first proportion is greater than the second proportion.

7. The ureteral stent according to claim 6, wherein
the first proportion is greater than the second proportion by 20 wt.% or more.

8. The ureteral stent according to claim 1, wherein
the first material is tungsten.

9. The ureteral stent according to claim 8, wherein
the first proportion is 50 wt.% or more.

10. The ureteral stent according to claim 1, wherein
the second material is barium sulfate.

11. The ureteral stent according to claim 10, wherein
the second proportion is from 10 wt.% or more to 30 wt.% or less.

12. The ureteral stent according to claim 1, wherein
the target curved part includes
a first section curved in a circular shape, and
a second section disposed inside a virtual circle along which the first section, and including an end part of the tubular body.

13. The ureteral stent according to claim 1, wherein
the target curved part is curved in a spiral shape,
a third section, of the target curved part, adjacent to the extension part, and a fourth section, of the target curved part, adjacent to the end part of the tubular body overlap each other in an extending direction of a spiral center, and
the first material is contained in section of the target curved part excluding the third section, and the second material is contained in the third section.

14. The ureteral stent according to claim 1, wherein
the target curved part is curved in a spiral shape,
a third section, of the target curved part, adjacent to the extension part, and a fourth section, of the target curved part, adjacent to the end part of the tubular body overlap each other in an extending direction of a spiral center, and
an X-ray transmittance of the third section is lower than an X-ray transmittance of the extension part and of the fourth section.

15. The ureteral stent according to claim 1, wherein
an X-ray transmittance of an adjacent part, of the target curved part, adjacent to the extension part is lower than an X-ray transmittance of the extension part.
